# EUROPEAN PATENT SPECIFICATION

(11) **EP 2 133 079 B1**
(45) Date of publication and mention of the grant of the patent: **05.10.2011**
(21) Application number: 08425421.8
(22) Date of filing: 12.06.2008
(51) Int. Cl.: A61K 31/473, A61K 31/343

(54) **Compositions for the treatment of vaginal infections with chronic inflammation**
Zusammensetzungen zur Behandlung von vaginalen Infektionen mit chronischer Entzündung
Compositions pour le traitement d'infections vaginales accompagnées d'inflammation chronique

(43) Date of publication of application: 16.12.2009
(73) Proprietor: Indena S.p.A., 20139 Milano (IT)
(72) Inventor: Bombardelli, Ezio, 27027 Gropello Cairoli (PV) (IT); Fontana, Gabriele, 20139 Milano (IT); Giori, Andrea, 20139 Milano (IT); Morazzoni, Paolo, 20139 Milano (IT); Riva, Antonella, 20139 Milano (IT); Ronchi, Massimo, 20139 Milano (IT)
(74) Representative: Minoja, Fabrizio

(56) References cited:
- EP-A- 0 464 297
- EP-B- 0 932 408
- WO-A-92/19242
- WO-A-2008/012666
- CARTER C A; CHAMBERLAIN K; WAIN R L: "Investigations on fungicides. XX. The fungitoxicity of analogues of the phytoalexin 2-(2'-methoxy-4'-hydroxyphenyl)-6-methoxyb enzofuran (vignafuran)" ANNALS OF APPLIED BIOLOGY, vol. 88, 1978, pages 57-64, XP002499176
- CHAMBERLAIN K; CARTER C A: "THE FUNGITOXICITY OF SUBSTITUTED 2-PHENYLBENZOFURANS" PESTICIDE SCIENCE, ELSEVIER APPLIED SCIENCE PUBLISHER. BARKING, GB, vol. 11, no. 5, 1 January 1980 (1980-01-01), pages 526-532, XP001074376 ISSN: 0031-613X
- PSOTOVA J; VERCERA R; ZDARILOVA A; ANZENBACHEROVA E; KOSINA P SVOBODOVA A; HRBAC J; JIROVSKY D; STIBOROVA M; LICHNOVSKY V ET AL: "Safety assessment of sanguiritrin, alkaloid fraction of Macleaya cordata, in rats" VETERINARNI MEDICINA, vol. 51, no. 4, 2006, pages 145-155, XP002499177

## Description

### Summary

The present invention relates to compositions based on benzofuran compounds and benzophenanthridine alkaloids, which possess anti-inflammatory, antibacterial and antifungal activity useful in the treatment of vaginal infections and the resulting inflammatory states.

### Prior art

Vaginitis is often initially asymptomatic, but with time can degenerate into infections which may be dangerous. Vulvovaginal infections, whether they are of viral, bacterial, fungal or protozoal origin (Herpes, trichomoniasis, candidiasis) cause vulvar itching, stinging, irritation and lesions, followed by external dysuria and vulvar dyspareunia. Vaginitis can lead to a series of serious events, with recurrent infections, such as toxicity to other organs and apparatus. This phenomenon is particularly important in many developing countries, where these events predispose the sufferer to the risk of contracting HIV or other sexually transmissible diseases.

Trichomoniasis presents symptoms such as a yellowish, purulent exudate with vulvar irritation, inflammation of the vaginal and vulvar epithelium, and petechial lesions of the cervix. The pH of the secretion is greater than 5, thus promoting the development of *Trichomonas*. In candidiasis there is severe vulvar itching with erythema and oedema, and the secretions are foul-smelling, as in the case of bacterial vaginitis. These disorders are treated with oral antibiotics and antifungals administered at high doses, or with gels for local treatment. These treatments always take a long time, and can have side effects.

The benzophenanthridine alkaloids isolated from *Macleaya cordata*, *Macleaya microcarpa, Sanguinaria canadensis* and *Chelidonia majus* are particularly active on strains directly involved in vaginal infections, such as *Trichomonas vaginalis, Escherichia coli, Pseudomonas aeruginosa* and the like.

According to the invention, the benzofuran compounds have the following formula 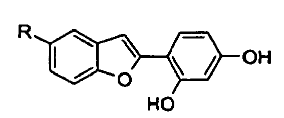 where R may be hydrogen or a linear or branched alkyl chain with 2 to 6 carbon atoms, or an alkyl chain substituted by amine, nitro groups; R is preferably hydrogen or alkyl C 1-C3.

Said benzofuran compounds are known and can be prepared by conventional methods, for example by reaction of a phenol suitably substituted with 2-phenoxy-2',4'-dimethoxyacetophenone in the conditions reported in Chimie Therapeutique 1973, 8, 398, followed by cyclisation in the presence of polyphosphoric acid in xylene and hydrolysis of the methoxy and hydroxy groups. The benzofuran compounds used in the compositions according to the invention have structural formula 1 and possess a powerful antibacterial and antifungal action against numerous strains of Candida.

### Description of the invention

The present invention relates to compositions based on:
a) benzophenanthridine alkaloids; and
b) benzofuran compounds;
   and possibly
c) extract of *Zanthoxylum bungeanum* or *Echinacea angustifolia*;
   which possess anti-inflammatory, antibacterial and antifungal activity useful in the treatment of vaginal infections and the resulting inflammatory states, especially vaginitis of various origins with associated inflammatory problems.

More particularly, the present invention relates to formulations based on:
a) benzophenanthridine alkaloids selected from sanguinarine and/or chelerythrine and/or derivatives thereof; and
b) benzofuran compounds as specified above;
   and possibly
c) extract of *Zanthoxylum bungeanum* or *Echinacea angustifolia.*

It has now surprisingly been found that the compositions according to the invention possess an antibacterial, antifungal and antienzymatic activity greater than that of the sum of the various components administered separately. Said effect may be due to a synergistic action mechanism which takes place between the various components of the association in question. The compositions according to the invention rapidly eliminate these infections, eliminating the presence of the saprophyte and reducing inflammation, itching and the vaginal pH.

According to the invention, the compositions will contain the various components in the following intervals (by weight per unit dose):
a) benzophenanthridine alkaloids: from 0.15 mg to 15 mg; and
b) benzofuran compounds: from 0.2 to 25 mg;
   and possibly
c) extract of *Zanthoxylum bungeanum* or *Echinacea angustifolia:* from 0.1 to 10 mg.

According to a particularly preferred aspect, the compositions in question will contain the various components within the following intervals (by weight per unit dose):
a) benzophenanthridine alkaloids: from 0.4 to 10 mg; and
b) benzofuran compounds: from 0.4 to 10 mg;
   and possibly
c) extract of *Zanthoxylum bungeanum* or *Echinacea angustifolia:* from 0.2 to 5 mg.

The benzophenanthridine alkaloids sanguinarine and chelerythrine may be present in free or salified form, as such in substantially pure form or in the form of extracts of *Sanguinaria canadensis, Macleaya cordata, Macleaya microcarpa or Chelidonia majus*. According to a preferred aspect, the benzophenanthridine alkaloids will be present in a form salified with luteic acid. Said salts, which are prepared by reacting the sulphates or chlorides of the alkaloids with the sodium or potassium salt of luteic acid and subsequent crystallisation, have proved particularly effective for the purposes of this invention. In particular, sanguinarine is a powerful anti-angiogenesis factor which helps to reduce inflammation (Jong-Pil Eun 2004). *In vivo*, sanguinarine suppresses capillary formation in Matrigel and in the chorioallantoic membrane in chicken embryo. (Jong-Pil Eun 2004).

Said benzophenanthridine alkaloids not only have considerable antibacterial, antifungal, and antitrichomonas activity, but also present considerable activity against cytomegalovirus and papillomavirus. For this reason the archetypes of these alkaloids, sanguinarine, chelerythrine and chelidonine, which also have an analgesic effect, are very useful in the treatment of vaginitis of different etiologies. These compounds act in synergy with one another to reduce inflammation, and consequently the symptoms, and to suppress the disorder.

The compounds with a benzofuran structure described above may be present as such or in the form of extracts containing them, such as extracts of *Krameria triandra, Eupomatia laurina* and *Piper sp*. The compounds isolated from *Krameria triandra* which have proved particularly active are Eupomatenoid 6 and neolignan 2-(2,4-dihydroxyphenyl)-5-(E)-propenyl-benzofuran, which have demonstrated antibacterial and antifungal activity on numerous strains of Gram+ bacteria, fungi and anaerobic bacteria.

According to a particularly preferred aspect, the compositions in question will also contain an extract of *Zanthoxylum bungeanum* or *Echinacea angustifolia* to help eliminate itching and/or pain, when present. This action is due to the presence of isobutylamides which bind the cannabinoid CB2 and CB1 receptors. The formulations according to the invention can be prepared according to well-known conventional methods, such as those described in "Remington's Pharmaceutical Handbook", Mack Publishing Co., N.Y., USA, together with suitable excipients.

The compositions according to the invention will be conveniently formulated in water/oil emulsions with other compatible excipients for external treatment of the anogenital region; for internal treatments the compounds will be suspended in oils in soft gelatin capsules which disintegrate easily after introduction into the vaginal meatus.

Examples of formulations according to the invention include creams, ointments, powders, lotions and the like, vaginal pessaries or equivalent formulations, including capsules that dissolve at internal body temperature.

The examples set out below illustrate the invention, without limiting its scope.

### Example 1 - Preparation of benzofuran compounds

### Stage A. Preparation of 2-phenoxy-2',4'-dimethoxyacetophenone (a)

A solution of 2-bromo-2',4'-dimethoxyacetophenone (5 g, 19.1 mmols) in 25 mL of 2-butanone was added to a suspension of phenol (1.8 g, 19.1 mmols), K₂CO₃ (2.6 g, 19.1 mmols) and Kl (41.5 mg, 0.25 mmols) in 20.0 mL of the same solvent. The solution was then refluxed for 20 hours. The mixture was filtered and the solvent was eliminated under vacuum. The residue obtained was dissolved in EtOAc and washed with a 10% aqueous solution of NaOH and then with water. The organic extract was dried over Na₂SO₄, filtered and evaporated under vacuum. Finally, the crude residue was washed with Et₂O and dried under low pressure to provide 4.4 g (yield: 84%) of the title compound.

### Stage B. Preparation of 2-(2',4'-dimethoxyphenyl)benzofuran (b)

12 g of polyphosphoric acid was added to a solution of the compound obtained at Stage A (4.4 g, 16.2 mmols) in 130.0 mL of xylene. The mixture was refluxed for 2 hours, and then left to cool at ambient temperature. The solution was then decanted and evaporated under low pressure. The resulting residue (3.7 g, yield: 90%) was used at the next stage without further purification.

### Stage C. Preparation of 2-(2',4'-dihydroxyphenyl)benzofuran (1)

A mixture of the compound prepared at Stage B (3.7 g, 14.5 mmols) and pyridine hydrochloride (11.1 g, 96.4 mmols) was heated to 225°C for 45 minutes. The red product formed was poured into 10% HCl. The mixture was washed repeatedly with EtOAc; the combined organic layers were dried over Na₂SO₄ and evaporated. The residue was purified by column chromatography (hexane/EtOAc= 7:3) to provide. The final compound was obtained with a yield of 41% (1.36 g) after crystallisation from benzene.

### Formulation example 1

### Oily suspension for soft gelatin capsules to be inserted in the vaginal meatus

| | |
|---|---|
| *Macleaya cordata* lipophilic extract (75%) | 10 mg |
| 2,4-Dihydroxyphenyl-3-benzofuran | 10 mg |
| Soya lecithin | 60 mg |
| Beeswax | 50 mg |
| Vegetable oil q.s. to | 800 mg |

### Formulation example 2

### Cream (oil-in-water emulsion)

| | |
|---|---|
| Extract of *Krameria triandra* | 0.4 g |
| *Macleaya cordata* alkaloid fraction | 0.4 g |
| *Zanthoxylum bungeanum* lipophilic extract | 0.2 g |
| Propylene glycol | 10.00 g |
| lsopropyl myristate | 5.00 g |
| Cetyl alcohol | 5.00 g |
| Polysorbate 80 | 3.00 g |
| Carbomer | 0.40 g |
| Methyl parahydroxy benzoate | 0.10 g |
| Propyl parahydroxy benzoate | 0.05 g |
| Purified water q.s. to | 100 g |

### Formulation example 3

### Vaginal pessary

| | |
|---|---|
| 2,4-Dihydroxyphenyl-3-benzofuran | 10 mg |
| *Macleaya* alkaloid fraction | 3 mg |
| Glycerides of fatty acids q.s. to | 2.0 g |

## Claims

1. Compositions based on:
a) benzophenanthridine alkaloids; and
b) benzofuran compounds of formula 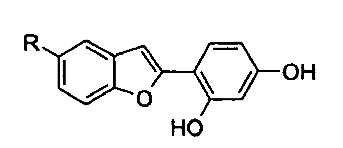 where R may be hydrogen or a linear or branched alkyl chain with 2 to 6 carbon atoms, or an alkyl chain substituted by amine, nitro groups;
and possibly
c) extract of *Zanthoxylum bungeanum* or *Echinacea angustifolia*.

2. Compositions as claimed in claim 1, based on:
a) benzophenanthridine alkaloids selected from sanguinarine, chelerythrine or chelidonine or derivatives thereof; and
b) benzofuran compounds as specified above;
and possibly
c) extract of *Zanthoxylum bungeanum* or *Echinacea angustifolia*.

3. Compositions as claimed in claims 1 and 2, wherein the various components are present in the following intervals (by weight per unit dose):
a) benzophenanthridine alkaloids: from 0.15 mg to 15 mg; and
b) benzofuran compounds: from 0.2 to 25 mg;
and possibly
c) extract of *Zanthoxylum bungeanum* or *Echinacea angustifolia:* from 0.1 to 10 mg.

4. Compositions as claimed in claim 3, wherein the various components are present in the following intervals (by weight per unit dose):
a) benzophenanthridine alkaloids: from 0.4 to 10 mg; and/or
b) benzofuran compounds: from 0.4 to 10 mg;
and possibly
c) extract of *Zanthoxylum bungeanum* or *Echinacea angustifolia*: from 0.2 to 5 mg.

5. Compositions as claimed in the preceding claims, wherein the benzophenanthridine alkaloids sanguinarine and chelerythrine are present in free or salified form, as such in substantially pure form or in the form of extracts of *Sanguinaria canadensis, Macleaya cordata, Macleaya microcarpa or Chelidonia majus.*

6. Compositions as claimed in claim 5, wherein the benzophenanthridine alkaloids are present in salified form with luteic acid.

7. Compositions as claimed in claims 1-4, wherein the benzofuran compounds are present as such or in the form of extracts containing them.

8. Compositions as claimed in claim 7, wherein the benzofuran compounds are present in the form of extracts of *Krameria triandra, Eupomatia laurina* and *Piper sp*.

9. Formulations as claimed in any one of claims 1 to 8, in the form of water/oil emulsions, soft gelatin capsules, vaginal pessaries or equivalent formulations, creams, ointments, powders, lotions, and the like.

10. The use of:
a) benzophenanthridine alkaloids; and
b) benzofuran compounds;
and possibly
c) extract of *Zanthoxylum bungeanum* or *Echinacea angustifolia*
for the preparation of topical formulations for the treatment of vaginal infections and the resulting inflammatory states.

11. The use as claimed in claim 10, wherein the vaginal infections are vaginitis of various origins with associated inflammatory problems.

## Patentansprüche

1. Zusammensetzungen, basierend auf:
a) Benzophenanthridin-alkaloiden; und
b) Benzofuran-Verbindungen der Formel 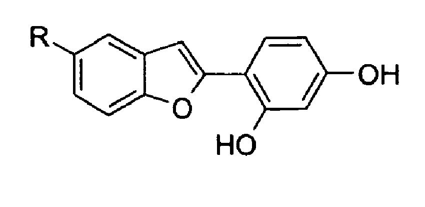 worin R Wasserstoff oder eine lineare oder verzweigte Alkyl-Kette mit 2 bis 6 Kohlenstoff-Atomen, oder eine Alkyl-Kette, substituiert mit Amin, Nitro-Gruppen, sein kann; und gegebenenfalls
c) Extrakt von *Zanthoxylum bungeanum* oder *Echinacea angustifolia.*

2. Zusammensetzungen nach Anspruch 1, basierend auf:
a) Benzophenanthridin-alkaloiden, ausgewählt aus Sanguinarin, Chelerythrin oder Chelidonin oder Derivaten davon; und
b) Benzofuran-Verbindungen, wie vorstehend ausgewiesen; und gegebenenfalls
c) Extrakt von *Zanthoxylum bungeanum* oder *Echinacea angustifolia.*

3. Zusammensetzungen nach Ansprüchen 1 und 2, worin die verschiedenen Komponenten in den nachstehenden Intervallen (als Gewicht pro Einheits-Dosis) vorliegen:
a) Benzophenanthridin-alkaloide: von 0,15 mg bis 15 mg; und
b) Benzofuran-Verbindungen: von 0,2 bis 25 mg; und gegebenenfalls
c) Extrakt von *Zanthoxylum bungeanum* oder *Echinacea angustifolia:* von 0,1 bis 10 mg.

4. Zusammensetzungen nach Anspruch 3, worin die verschiedenen Komponenten in den nachstehenden Intervallen (als Gewicht pro Einheits-Dosis) vorliegen:
a) Benzophenanthridin-alkaloide: von 0,4 bis 10 mg; und/oder
b) Benzofuran-Verbindungen: von 0,4 bis 10 mg; und gegebenenfalls
c) Extrakt von *Zanthoxylum bungeanum* oder *Echinacea angustifolia:* von 0,2 bis 5 mg.

5. Zusammensetzungen wie in den vorangehenden Ansprüchen, worin die Benzophenanthridin-alkaloide Sanguinarin und Chelerythrin in freier oder Salz-Form, als solche in im Wesentlichen reiner Form oder in Form von Extrakten von *Sanguinaria canadensis, Macleaya cordata, Macleaya mic*rocarpa oder *Chelidonia majus* vorliegen.

6. Zusammensetzungen nach Anspruch 5, worin die Benzophenanthridin-alkaloide in Salz-Form mit Luteinsäure vorliegen.

7. Zusammensetzungen nach Ansprüchen 1-4, worin die Benzofuran-Verbindungen als solche oder in Form von dieselben enthaltenden Extrakten vorliegen.

8. Zusammensetzungen nach Anspruch 7, worin die Benzofuran-Verbindungen in Form von Extrakten von *Krameria* tri*andra, Eupomatia laurina* und *Piper sp.* vorliegen.

9. Formulierungen nach einem der Ansprüche 1 bis 8, in Form von Wasser / Öl-Emulsionen, Weichgelatine-Kapseln, Vaginal-Pessarien oder äquivalenten Formulierungen, Cremes, Salben, Pulvern, Lotionen und dergleichen.

10. Verwendung von:
a) Benzophenanthridin-alkaloiden; und
b) Benzofuran-Verbindungen; und gegebenenfalls
c) Extrakt von *Zanthoxylum bungeanum* oder *Echinacea angustifolia*
zur Herstellung von topischen bzw. örtlichen Formulierungen für die Behandlung von Vaginal-Infektionen und den sich ergebenden entzündlichen Zuständen.

11. Verwendung nach Anspruch 10, worin die Vaginal-Infektionen Vaginitis von verschiedenem Ursprung mit zugehörigen entzündlichen Problemen darstellen.

## Revendications

1. Compositions basées sur :
a) des alcaloïdes de benzophénanthridine ; et
b) des composés de benzofuranne de formule 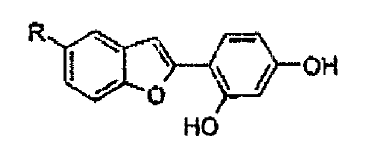 dans laquelle R peut être un atome d'hydrogène ou une chaîne d'alkyle linéaire ou ramifiée avec 2 à 6 atomes de carbone, ou une chaîne d'alkyle substituée par des groupes amine, nitro ;
et le cas échéant
c) un extrait de *Zanthoxylum bungeanum* ou *d'Echinacea angustifolia.*

2. Compositions selon la revendication 1, basées sur :
a) des alcaloïdes de benzophénanthridine choisis parmi de la sanguinarine, de la chélérythrine ou de la chélidonine ou leurs dérivés ; et
b) des composés de benzofuranne tels que spécifiés ci-dessus ;
et le cas échéant
c) un extrait de *Zanthoxylum bungeanum* ou *d'Echinacea angustifolia.*

3. Compositions selon les revendications 1 et 2, dans lesquelles les divers composants sont présents dans les intervalles suivants (en poids par dose unitaire) :
a) alcaloïdes de benzophénanthridine : de 0,15 mg à 15 mg ; et
b) composés de benzofuranne : de 0,2 à 25 mg ;
et le cas échéant
c) extrait de *Zanthoxylum bungeanum* ou d'*Echinacea angustifolia* : de 0,1 à 10 mg.

4. Compositions selon la revendication 3, dans lesquelles les divers composants sont présents dans les intervalles suivants (en poids par dose unitaire) :
a) alcaloïdes de benzophénanthridine : de 0,4 à 10 mg ; et/ou
b) composés de benzofuranne : de 0,4 à 10 mg ;
et le cas échéant
c) extrait de *Zanthoxylum bungeanum* ou *d'Echinacea angustifolia* : de 0,2 à 5 mg.

5. Compositions selon les revendications précédentes, dans lesquelles les alcaloïdes de benzophénanthridine, la sanguinarine et la chélérythrine, sont présents dans une forme libre ou salifiée, telle que dans une forme essentiellement pure ou bien sous la forme d'extraits de *Sanguinaria canadensis,* de *Macleaya cordata,* de *Macleaya microcarpa* ou de *Chelidonia majus.*

6. Compositions selon la revendication 5, dans lesquelles les alcaloïdes de benzophénanthridine sont présents dans une forme salifiée avec de l'acide lutéique.

7. Compositions selon les revendications 1 à 4, dans lesquelles les composés de benzofuranne sont présents tels que ou bien sous la forme d'extraits les contenant.

8. Compositions selon la revendication 7, dans lesquelles les composés de benzofuranne sont présents sous la forme d'extraits de *Krameria triandra,* d'*Eupomatia laurina* et de *Piper sp.*

9. Formulations selon l'une quelconque des revendications 1 à 8, sous la forme d'émulsions eau dans l'huile, de capsules molles, de pessaires vaginaux ou formulations équivalentes, de crèmes, de pommades, de poudres, de lotions, et analogues.

10. Utilisation de :
a) alcaloïdes de benzophénanthridine ; et
b) composés de benzofuranne ;
et le cas échéant
c) extrait de *Zanthoxylum bungeanum* ou *d'Echinacea angustifolia.*
pour la préparation de formulations topiques destinées au traitement d'infections vaginales et des états inflammatoires qui en résultent.

11. Utilisation selon la revendication 10, dans laquelle les infections vaginales sont des vaginites d'origines diverses avec des problèmes inflammatoires associés.
